# EUROPEAN PATENT SPECIFICATION

(11) **EP 2 437 058 B1**
(45) Date of publication and mention of the grant of the patent: **16.08.2017**
(21) Application number: 10780718.2
(22) Date of filing: 06.05.2010
(51) Int. Cl.: G01N 33/558, G01N 33/543

(54) **METHOD FOR SIGNAL AMPLIFICATION DURING LATERAL-FLOW ANALYSIS**
VERFAHREN ZUR SIGNALVERSTÄRKUNG WÄHREND DER SEITENFLUSSANALYSE
PROCÉDÉ D'AMPLIFICATION DU SIGNAL LORS D'UNE ANALYSE SUR MEMBRANE

(30) Priority: 28.05.2009 KR 20090047004
(43) Date of publication of application: 04.04.2012
(73) Proprietor: OSANG HEALTHCARE CO., LTD., Anyang-si, Gyeonggi-do (KR)
(72) Inventor: BAE, Byeong-Woo, Kyunggi 431-080 (KR); LEE, Sung-Dong, Kyunggi 431-080 (KR); KIM, Min-Gon, Daejeon 305-759 (KR); SHIN, Youn-Beom, Daejeon 302-772 (KR); JANG, Jin-Hee, Kyunggi 431-080 (KR); SHIN, Ji-Hun, Kyunggi 431-080 (KR); LEE, Seok-Ki, Kyunggi 431-080 (KR)
(74) Representative: D Young & Co LLP
(86) International application number: PCT/KR2010/002854
(87) International publication number: WO 2010/137807

(56) References cited:
- EP-A1- 2 042 871
- KR-A- 20080 069 058
- US-A1- 2004 191 760
- US-A1- 2004 265 922
- US-A1- 2007 020 768
- US-A1- 2007 087 453
- US-A1- 2008 233 659
- JASDEEP KAUR ET AL: "Immunochromatographic Dipstick Assay Format Using Gold Nanoparticles Labeled Protein-Hapten Conjugate for the Detection of Atrazine", ENVIRONMENTAL SCIENCE & TECHNOLOGY, vol. 41, no. 14, 1 July 2007 (2007-07-01), pages 5028-5036, XP055047538, ISSN: 0013-936X, DOI: 10.1021/es070194j
- ZHANFANG MA ET AL: "Naked-eye sensitive detection of immunoglubulin G by enlargement of Au nanoparticles in vitro.", ANGEWANDTE CHEMIE INTERNATIONAL EDITION, vol. 41, no. 12, 1 June 2002 (2002-06-01), pages 2176-2179, XP55047202, ISSN: 1433-7851
- SU ET AL: "Immunoassay signal amplification on glass slides based on electroless deposition", COLLOIDS AND SURFACES. B, BIOINTERFACES, ELSEVIER, AMSTERDAM, NL, vol. 44, no. 2-3, 1 August 2005 (2005-08-01), pages 99-103, XP027803452, ISSN: 0927-7765 [retrieved on 2005-08-01]
- JOURNAL OF IMMUNOLOGICAL METHODS vol. 312, 2006, pages 27 - 33, XP025158167
- ANAL. BIOANAL. CHEM. vol. 393, 13 August 2008, pages 569 - 582, XP019652854
- SENSOR vol. 8, 2008, pages 5535 - 5559, XP008149209

## Description

### [Technical Field]

The present invention relates to a signal amplifying method in a lateral flow analysis for detecting an analyte with high sensitivity and a lateral flow analysis device using the same. In more detail, the present invention relates to a signal amplifying method in a lateral flow analysis with high sensitivity, in which gold ions and a reductant are added and react to a seed of gold nano particles to amplify a signal, and a lateral flow analysis device using the same.

### [background Art]

An immunochromatographic assay is a method for analyzing an analyte qualitatively and quantitatively in a short period by using characteristics that biological materials or chemical materials are specifically attached to each other. Especially, according to a sandwich immunoassay, a first antibody, which is specifically combined with a first epitope of an analyte (i.e., a target of an existence and concentration test), is fixed to a solid supporter, and a specific second antibody is used for a second epitope of the analyte.

As a device for the above analysis, an analysis strip or an analysis device including an analysis strip mounted and assembled in a housing of the device is generally used. At this point, if a fluid including an analyte is applied to one side of a porous strip, an analyte is attached to an antibody including a marker and a fixed antibody according to an analysis method while the fluid flows through a capillary phenomenon.

In more detail, in relation to a lateral flow device, an antibody is typically fixed at a porous membrane where a liquid seed flows through a capillary phenomenon; a sample pad and a bonding pad are disposed on the upper stream of the membrane; and an absorbing pad is disposed on the lower stream of the membrane. The sample pad absorbs the liquid seed including an analyte and also guarantees a uniform flow. A marker having an antibody attached, which is selectively combined with an analyte, is dried in the bonding pad. A fixed antibody, which is selectively combined with an analyte, and a material, which is combined with an antibody fixed at a marker, are fixed at the respectively different positions of the membrane, thereby forming a detection site and a control site. An antibody fixed at the membrane, which is selectively combined with the analyte, and an antibody, which is fixed at the marker, may be configured to be combined with the analyte in a sandwitch form. The absorbing pad is formed of a material for absorbing a liquid seed. Like this, in relation to an immunochromatographic assay device, when a liquid seed including an analyte is dropped in a sample pad, an antibody-marker having selectivity with respect to the analyte and an antibody fixed at the membrane are combined in a sandwich form. As a result, a band, which may be confirmed with the naked eye, is formed at the position of the membrane having the antibody fixed.

As a typical technique, an immunochromatographic signal amplifying method, in which after a first conjugate is combined with an antigen and then a second conjugate is additionally combined with the antigen, they are finally coupled to an antibody fixed at a membrane, is disclosed. However, a typical immunochromatographic assay may have limitations in measuring a seed that requires high sensitivity. Furthermore, a method of growing gold nano particles by a reaction of gold ions and a reductant may be disclosed in the reporter of other researchers (Angew. Chem. Int. Ed. 2002, 41, 2176-2179; Colloids and Surfaces B: Biointerfaces, 2005, 44, 99-103). However, a technical idea that the growth of gold nano particles is used for a signal amplifying method of an immunochromatographic sensor or an additional pad including gold ions and a reductant is mounted on an immunochromatographic device to easily obtain signal amplification is not known yet.

EP 2 042 871 discloses a method for mixing two or more types of liquids in a porous carrier. The method involves a device comprising a detection zone and a control zone. The detection zone comprises both an analyte and organic silver salts and reducing agents.

Jasdeep et al ("Immunochromatographic Dipstick Assay Format Using Gold Nanoparticles Labeled Protein-Hapten Conjugate for the Detection of Atrazine", Environmental Science and Technology, vol. 41, no.14, 1 July 2007, pages 5028 to 5036) discloses a lateral-flow-based dispstick immunoassay format using a hapten-protein-gold conjugate for the rapid screening of atrazine in water.

Accordingly, the inventors of the present invention provide a method of improving the sensitivity of signal amplification and a lateral flow analysis using the same. In relation to the method, a conjugate, which has a first antibody or a first specific combining material and gold nano particles combined therewith, and an analyte are combined. Then, the combined result is combined with a fixed antibody or specific combining material through sandwich reaction. Then, gold ions are reduced through the reaction of gold ions and reductant. After that, gold nano particles are formed by using the gold ions as a seed.

### [Disclosure]

### [Technical Problem]

The present invention provides a signal amplifying method in a lateral flow analysis.

The present invention also provides a lateral flow analysis device having improved sensitivity through the lateral flow analysis.

### [Technical Solution]

According to an aspect of the present invention, a signal amplifying method in a lateral flow analysis includes: providing a liquid sample including analyte in a sample pad, combining a conjugate with an analyte, the conjugate including a first antibody or a first specific binding material and gold nanoparticles, wherein the first antibody or a first specific binding material is specifically combined with a first epitope or a first binding portion (ligand) of the analyte; combining the analyte and combined conjugate with a fixed second antibody or second specific combining material, which is specifically combined with a second epitope or a second binding portion (ligand) of the analyte; and adding gold ions and a reductant to cause signal amplification reaction by applying pressure to the pad for signal amplification including gold ions and a reductant which is disposed between a first position which is not in contact with a basic strip and a second position which is in contact with the basic strip, and
wherein the pad for signal amplification is selectively contacted to at least one of the bonding pad and membrane, when the pad for signal amplification is moved from the first position to the second position by pressure applied to the pad for signal amplification.

According to still another aspect of the present invention, a lateral flow analysis device includes: a basic strip comprising: a bonding pad including a conjugate having a first antibody or a first specific binding material and gold nano particles, wherein the first antibody or the first specific binding material of the conjugate is capable of being specifically combined with a first epitope or a first binding portion (ligand) of an analyte,;
a membrane having a detection site including a fixed second antibody or second specific binding material, wherein the fixed second antibody or the second specific binding material is capable of being specifically combined with a second epitope or a second binding portion of the analyte; and a pad for signal amplification including gold ions and a reductant, wherein the pad for signal amplification is disposed between a first position which is not in contact with the basic strip and a second position which is in contact with the basic strip, and
wherein the pad for signal amplification is selectively contacted to at least one of the bonding pad and the membrane, when the pad for signal amplification is moved from the first position to the second position by pressure applied to the pad for signal amplification.

### [Advantageous Effects]

According to the signal amplifying method, a conjugate and an analyte are fixed at a capture antibody or a specific combining material, and subsequently, a reaction liquid including gold ions and a reductant are added to cause reaction. As a result, excellent signal amplification effects may be provided, and also, a lateral flow analysis device having improved sensitivity may be manufactured through the signal amplifying method.

### [Description of Drawings]

Fig. 1 is a schematic view when a signal is amplified during an antigen analysis through a method according to the present invention. Reference numerals 1, 2, 3, 4, 5, and 6 represent a first antibody, a gold nano particle, a conjugate, an analyte, a second antibody, and a gold nano particle formed when gold ions are reduced, respectively.
Fig. 2(a) is a schematic view illustrating a structure of each pad during a lateral flow analysis device according to the present invention. Reference numberals 10, 11, 12, 13, 14, 15, and 16 represent a sample pad, a bonding pad, a membrane, a detection site, a control site, an absorbing pad, and a pad for signal amplification including gold ions and a reductant, respectively.
Fig. 2(b) is a schematic view illustrating a structure after a pad is disposed in a lateral flow analysis device.
Fig. 2(c) is a sectional view illustrating a lateral flow analysis device where the pad for signal amplification 16 remains not contacting a basic strip of an analysis device.
Figs. 3 and 4 are views illustrating experimental results of signal amplification effects according to the reduction of gold ions by using myoglobin.
Figs. 5 and 6 are views illustrating exterimetal results of signal amplification effects in an immune chromatography by using myoglobin.

### [Best Mode]

According to a signal amplifying method of the present invention, in relation to an immune chromatography, a conjugate including a first antibody and gold nano particles is combined with an antigen and then is combined with a second antibody (i.e., a fixed capture antibody) through a sandwich reaction. Subsequently, gold ions and a reductant are added. That is, according to the signal amplifying method of the present invention, a conjugate including a first antibody or a first specific binding material, and gold nano particles is combined with an analyte, and then, is combined with a fixed second antibody (i.e., a fixed capture antibody) or fixed second specific combining material. Subsequently, after gold ions and a reductant are added to cause reaction, Au(III) ions around the gold nano parcels are reduced by using the gold nano particles as a seed. As a result, strong color is represented. Due to this, a signal is amplified.

The signal amplifying method may be applied to a lateral flow analysis method. The lateral flow analysis method may include a vertical flow or flow through method. In the case of the vertical flow method, a seed is stacked vertical to porous layers disposed in parallel.

Moreover, the lateral flow method is not limited to the antibody-antigen reaction. A combining portion (Ligand)' mentioned in the present invention includes a combining portion or protein Ligand and nucleic acid molecule (DNA or RNA) sequence, and a specific combining material' includes biomolecules such as protein, virus phage, nucleic acid molecule (Aptamer), and hapten (DNP). The present invention is not limited thereto.

The 'conjugate' mentioned in the present invention includes a first antibody or a first specific combining material, and gold nano particles. The conjugate flows through a strip after combining with an analyte, and then, is fixed at a capture second antibody or a second specific combining material.

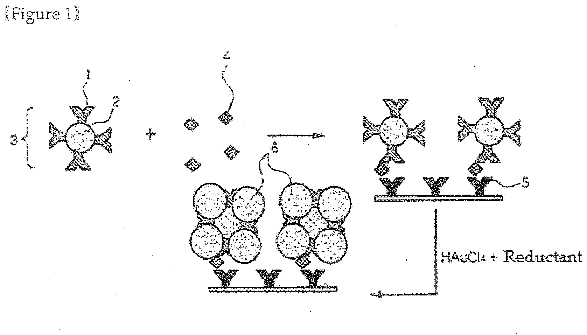

The mechanism of a signal amplifying method in a lateral flow analysis will be described in more detail with reference to Fig. 1. Fig. 1 is a view when an antigen-antibody reaction is used in a lateral flow analysis. As shown in Fig. 1, a conjugate 3 including a first antibody 1, which is combined specifically to a first epitope of an analyte, and a gold nano particle 2, which combined with the first antibody 1, is combined with an analyte. And, the analyte combined with the conjugate 3 flows along a strip and is combined with a fixed second antibody 5, which is specifically combined with a second epitope of the analyte. After these sandwich combinations, gold ions react to a reductant. As a result, a signal is amplified during a lateral flow analysis by the gold nano particle 6, which is formed additionally by using the gold nano particle 2 as a seed.

The gold ions used for the present invention are used as a precursor of the gold nano particle. If the gold ions include Au(III), it is not limited to the present invention. The gold ions may include HAuCl₄.

A reductant used for the present invention may include citric acid, ascorbic acid, sodium borohydride, hydroxymethyl, and phosphonium chloride. The reductant may include a mixture of at least two reductants. More preferably, the reductant may include hydroxylamine and citric acid. The citric acid may be used as the reductant and also may prevent nano particles from entanglement after being attached to the surface of gold nano particles.

An analyte detected through the method of the present invention is not limited to being combined with a first antibody and a second antibody through an immunological reaction, i.e., an antigen-antibody reaction, and forming a sandwich-immune complex. For example, the analyte may be applied to protein or DNA, pollutants such as endocrine disruptor, and virus.

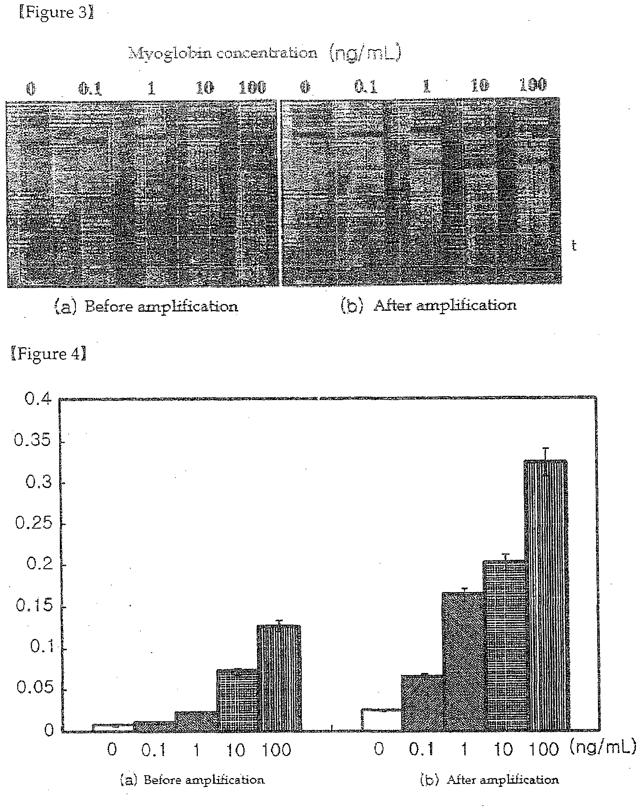

According to the present invention, if an antigen and an antibody are materials combined with an analyte through an antigen-antibody reaction, they may be not limited to the present invention. If an analyte is an antibody, a material, which is specifically combined with the analyte, may be used as an antigen. The antigen and antibody may include well known antigens and antibodies according to an analyte. Furthermore, the reaction of a 'specific combining material', which recognizes an analyte as a 'combining portion (ligand)' and is selectively combined thereto, may be included in an antigen-antibody reaction in a broader sense.
A signal amplification effect from the reduction of gold ions by using myoglobin as an analyte will be confirmed in an embodiment 3. As a result, as shown in Fig. 4, it is confirmed that sensitivity is increased at least ten times than when a signal is amplified.

The lateral flow analysis device of the present invention uses the signal amplifying method, and includes a sample pad, a bonding pad, a pad for signal amplification, a porous membrane, and an absorbing pad. The sample pad receives a liquid seed including an analyte. The bonding pad includes a conjugate having a first antibody or a first specific combining material, which is specifically combined with a first epitope or a first combining portion (i.e., ligand), and gold nano particles, which are combined with the first antibody or the first specific combining material. The porous membrane includes a detection site having a fixed second antibody or second specific combining material, which is specifically combined with a second epitope or a second combining portion of the analyte having the conjugate combined, and a control site for error confirmation. The absorbing pad absorbs a liquid seed through a capillary phenomenon.

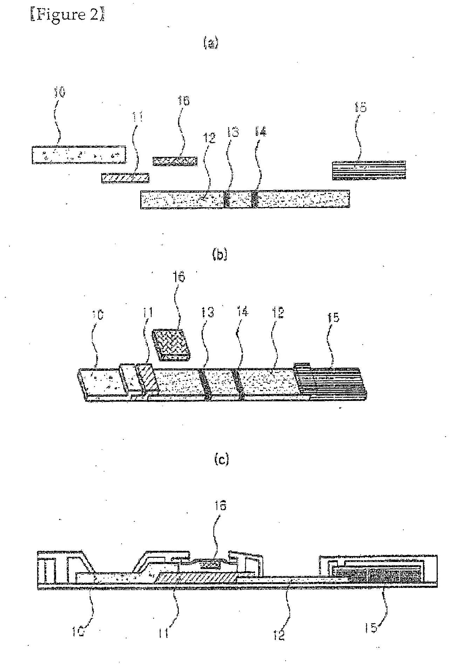

Fig. 2(a) is a schematic view illustrating a structure of each pad during a lateral flow analysis device according to the present invention. Reference numerals 10, 11, 12, 13, 14, 15, and 16 represent a sample pad, a bonding pad, a membrane, a detection site, a control site, an absorbing pad, and a pad for signal amplification including gold ions and a reductant, respectively. Fig. 2(b) is a schematic view illustrating a structure after a pad is disposed in a lateral flow analysis device. Fig. 2(c) is a sectional view illustrating a lateral flow analysis device where the pad for signal amplification 16 remains not contacting a basic strip (including the sample pad, the bonding pad, and the membrane) of an analysis device.

A pad used for manufacturing a lateral flow analysis device of the present invention is formed of a porous material including natural or synthetic material. The pad may include nitrocellulose.

The sample pad may be a portion into which a liquid seed including an analyte is absorbed first, and may be formed of one end of the membrane as it is or an additional unit. Also, the sample pad absorbs a seed including an analyte and transfers the analyte to the bonding pad through a capillary phenomenon. Additionally, according to an embodiment, if there is no sample pad, a seed including a target analyte is directly applied to the bonding pad.

In relation to the bonding pad, a conjugate is in a dry state and obtains fluidity to transfer to the membrane when a liquid seed including an analyte is absorbed. The bonding pad may be formed of glass fiber and cellulose.

The pad for signal amplification may be formed of the same material as or a different material from the bonding pad. Gold ions and a reductant are applied and dried on the pad for signal amplification. The gold ions and reductant may be simultaneously or separately applied and dried on the pad for signal amplification. If they are separately applied and dried, a plurality of pads for signal amplification may be used. The pad for signal amplification remains not contacting the sample pad, bonding pad, and membrane of the lateral flow analysis device. After a liquid seed including an analyte is absorbed in the absorbing pad, and then a conjugate including a first antibody and gold nano particles is combined with an antigen, in order to be combined with a second antibody (i.e., a fixed capture antibody) through a sandwich reaction. Subsequently, since the combined result contacts at least one of the bonding pad and the membrane in the lateral flow analysis device, gold ions and a reductant react to each other. Thus, Au(III) ions around the gold nano particles are reduced by using the gold nano particles as a seed. As a result, a signal is amplified.

Accordingly, the pad for signal amplification does not contact the basic strip of the lateral flow analysis device. For example, the pad for signal amplification is attached to an external case, not contacting the basic strip of the lateral flow analysis device. The pad for signal amplification may manually contact the basic strip of the lateral flow analysis device when a predetermined time elapses after a seed is put in the sample pad. Or, the pad for signal amplification may contact the basic strip of the lateral flow analysis device when an automatic device applies pressure. However, the pad for signal amplification may be attached to a case of the lateral flow analysis device with any form, in which the pad for signal amplification, which remains in no contact, may actively or passively contact the basic strip of the lateral flow analysis device when a predetermined time elapses after a seed is added. The present invention is not limited to a specific attachment method. The pad for signal amplification may be manufactured in various forms. For example, as shown in Fig. 2(c), when the both ends of the pad for signal amplification may be attached to the case of the lateral flow analysis device and then pressure is applied, the pad for signal amplification may contact the basic strip of the lateral flow analysis device. Additionally, the pad for signal amplification, which remains in no contact, may automatically contact the basic strip by an additional measuring device when a predetermined time elapses.

Since the pad for signal amplification is required to contact the basic strip sequentially after a conjugate including a first antibody or a first specific combining material and gold nano particles is combined with a second antibody (i.e., a fixed capture antibody) or a second specific combining material, it may contact the basic strip of the lateral flow analysis device after about 1 min to about 10 min (preferably, about 3 min to about 7 min) when a seed is added. However, the present invention is not limited to the time range. For example, when the pore size of the basic strip of the lateral flow analysis device is large, since reaction is completed in a short period, the pad for signal amplification may contact the basic strip in a shorter time than after a seed is introduced. That is, according to various cases, a time to contact the basic strip may vary.

When the pad for signal amplification contacts the basic strip of the lateral flow analysis device from the beginning, it may contact between the bonding pad and the membrane. Additionally, the pad for signal amplification may be disposed for contact on the bonding pad. Additionally, the pad for signal amplification may be disposed for contact on the membrane. Moreover, the pad for signal amplification may be disposed for contact on the bonding pad and the membrane.

Any membrane may be used only if it may provide the fluidity of a liquid seed, and may be formed of a porous layer. In more detail, a hydrophobic porous layer capable of adjusting a size of a predetermined micro pore such as nitrocellulose, cellulose, Poly-vinylidene fluoride (PVDF), poly(ethylene terephthalate (PET), polyethersulfone (PES), glass fiber, and nylon may be used to fix an antibody or a specific combining material and enzyme protein, and minimize nonspecific reactions. The membrane includes a detection site and a control site. The detection site has a second antibody or a second specific combining material, which is combined specifically to a second portion of the analyte having a conjugate combined. The control site as a control group according thereto detects whether there is abnormal reaction. The detection site is a portion representing a result for reading a test result. The control site is configured to confirm an error of the gold nano particle conjugate and a capture antibody or a fixed second specific combining material, and also confirm whether materials having mobility are reacted well until the detection site/control site without errors.

Any absorbing pad, which sufficiently absorbs residue after reaction through a capillary phenomenon, may be used, and for example, may include cellulose, cotton, and hydrophilic porous polymer.

Hereinafter, the present invention will be described in more detail through embodiments. The embodiments are merely for exemplifying the present invention, and the scope of protective rights of the present invention shall not construed as being limited by the embodiments.

### [Mode for Invention]

### Embodiment 1: synthesis of gold nano particle-antibody conjugate

0.1 mL of 0.1 M Borate buffer (pH 8.5) was added to 1 mL of a gold nano particle colloid solution (BBInternational, 20 nm), and then, 10 µL of 1 mg/mL first antibody was added to cause reaction for about 30 min. After the reaction, 0.1 mL of a solution obtained by dissolving 1%(w/v) Bovine Serum Albumin (BSA) (Sigma) in Phosphate Buffered Saline (PBS) was added to cause reaction for about 15 min at a room temperature. After the reaction, the process of centrifugation was used under conditions such as 10,000 rpm, 4 °C, and 20 min, and then, 1 mL of a BSA (Sigma) solution dissolved with 1 mg/mL concentration was added to 10 mM PBS over three times for purification and then was retrieved. The first antibody may use M012607(Fitzgerald) during an immune analysis for myoglobin.

### Embodiment 2: preparing immune chromatography

After a nitrocellulose membrane (Millipore, 180 sec Nitrocellulose) and an absorbing pad (Millipore) were attached to a plastic pad (Millipore), a dispenser system (Zeta Co.) drew a line on a membrane at 6 cm/sec to form a detection line and a control line by using a 1 mg/mL solution containing a capture antibody (i.e., a second antibody) dissolved in PBS and a 1 mg/mL solution containing a Goat antimouse IgG antibody (Sigma, M8642) dissolved in PBS as a control. After the membrane was dried and put in a cutter to be cut by a 3 mm interval. The second antibody, i.e., the capture antibody, may use a myoglobin capture antibody (Fitzgerald) for an immune analysis for myoglobin. After a bonding pad (GFC, Millipore Co.) was cut by 5x3 mm, 5 µL of the conjugate prepared in the embodiment 1 was applied and dried for use. After a sample pad was impregnated in a 1 % BSA, 0.5% Tween 20, 5% sucrose, 5% textran, 0.05% sodium azide aqueous solution and was dried, it is cut by 10×3 mm. A bonding pad and a sample pad ware attached to a plastic pad including the membrane and the absorbing pad as shown in Fig. 2(b).

### Embodiment 3: confirming signal amplification effects according to reduction of gold ions.

The immune chromatographic sensor assembled in the embodiment 2 was impregnated in a 96 well plate where 70 µL of PBS obtained by dissolving a myoglobin antigen with concentrations of 0ng/mL, 0.1ng/mL, 1ng/mL, 10ng/mL and 100 ng/mL was impregnated. If a signal was not amplified, the impregnated time was 10 min, and if a signal was amplified, after 5 min of impregnation, the immune chromatographic sensor was impregnated in 50 µL of a citrate buffer solution (5 mM, pH 4.0) obtained by dissolving 50 mM HAuCl₄ and 10 mM HONH₂. Then, after 5 min, a signal was observed. The measured result may be confirmed in Figs. 3 and 4. Fig. 3 represents a strip after the experiment and Fig. 4 represents a chart of the result. As a result, sensitivity was increased more than ten times compared to when a signal was not amplified.

### Embodiment 4: signal amplification effect according to reduction of gold ions using pad for signal amplification

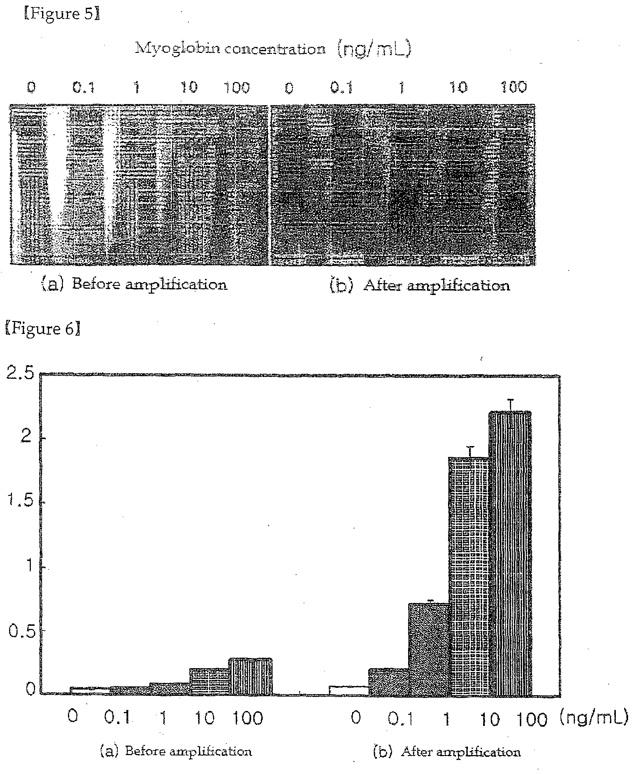

The immune chromatographic sensor assembled in the embodiment 2 was impregnated in a 96 well plate where 70 µL of PBS obtained by dissolving a myoglobin antigen with concentrations of 0ng/mL, 0.1ng/mL, 1ng/mL, 10ng/mL and 100 ng/mL was impregnated. If a signal was not amplified, the impregnated time was 10 min. If a signal was amplified, after 5 min, a pad for signal amplification was put on a bonding pad while the immune chromatographic sensor was impregnated. 10 ul of 250 mM HAuCl₄ was applied on a pad obtained by cutting Fusion 5 (Whatman) by 5x3 mm and 10 µL of a citrate buffer solution (25 mM, pH 4.0) obtained by dissolving 25 mM HONH₂ was applied on another Fusion 5 (Whatman) and then dried to prepare a pad for signal amplification. The measured result may be confirmed in Figs. 5 and 6. Fig. 5 represents a strip after the experiment and Fig. 6 represents a chart of the result. As a result, sensitivity is increased more than five times compared to when a signal was not amplified.

The present invention may, however, be embodied in different forms and should not be construed as limited to the embodiments. The present invention is defined by scopes of claims. Various modification and changes are possible for those skilled in the art. Hereinafter, several modification items are exemplarily disclosed.

For example, although not shown in the drawings, according to an analysis device including a sample pad (optional) 10, a bonding pad 11, a membrane 12, and an absorbing pad (optional) 15 in some cases, a solution for signal amplification including gold ions and a reductant may be applied to a sample pad or a binding pad when a predetermined time elapses after a seed including an analyte reacts in a test line of the membrane 12 with the device moving laterally. In this case, a solution including gold ions and a reductant in an additional container and the analysis device are provided as a set. Additionally, in a case of quantitative measurement instead of qualitative measurement, a measuring device having a reading unit for reading a test line 13 may allow the pad for signal amplification 16, which remains in no contact, to contact at least one of the bonding pad and the membrane when a predetermined time elapses after a seed is added. In this case, the measuring device includes a unit for contacting the pad for signal amplification. Therefore, gold ions in the pad for signal amplification may move toward the test line 13 through diffusion flow. Additionally, a user may manually apply pressure on a portion of a housing of the measuring device, thereby allowing a pad for signal amplification, which normally remains in no contact, to contact on at least one of the bonding pad and the membrane when a predetermined time elapses after a seed is added. Moreover, there may be a pad for signal amplification, which contacts on a basic strip or inserted into the middle of the basic strip from the beginning when it is manufactured. Lastly, a measuring device having a reading unit for measuring a result of a test line of an analysis device may include a storage unit for storing the solution for signal amplification and a unit for applying the solution on the bonding pad or the membrane when a predetermined time elapses after an analysis seed is added. These various methods and structures may be included in the technical ideas of the claims below.

### [Industrial Applicability]

The present invention may be used to measure biometric data.

## Claims

1. A signal amplifying method in a lateral flow analysis, the method comprising:
providing a liquid sample including analyte in a sample pad;
combining a conjugate with the analyte,
the conjugate including a first antibody or a first specific binding material and gold nano particles; wherein the first antibody or a first specific binding material is specifically combined with a first epitope or a first binding portion (ligand) of the analyte;
combining the analyte and combined conjugate with a fixed second antibody or second specific binding material via a second epitope or a second binding portion (ligand) of the analyte; and
adding gold ions and a reductant to cause signal amplification reaction by applying pressure to the pad for signal amplification including gold ions and a reductant which is disposed between a first position which is not in contact with a basic strip and a second position which is in contact with the basic strip, and
wherein the pad for signal amplification is selectively contacted to at least one of the bonding pad and the membrane, when the pad for signal amplification is moved from the first position to the second position by pressure applied to the pad for signal amplification.

2. A lateral flow analysis device comprising:
a basic strip comprising:
a bonding pad including a conjugate having a first antibody or a first specific binding material and gold nano particles, wherein the first antibody or the first specific binding material of the conjugate is capable of being specifically combined with a first epitope or a first binding portion (ligand) of an analyte; and
a membrane having a detection site including a fixed second antibody or a second specific binding material, wherein the fixed second antibody or the second specific binding material is capable of being specifically combined with a second epitope or a second binding portion (ligand) of the analyte;
a pad for signal amplification including gold ions and a reductant, wherein the pad for signal amplification is disposed between a first position which is not in contact with the basic strip and a second position which is in contact with the basic strip, and
wherein the pad for signal amplification is selectively contacted to at least one of the bonding pad and the membrane, when the pad for signal amplification is moved from the first position to the second position by pressure applied to the pad for signal amplification.

3. The device according to claim 2, further comprising a sample pad to which a liquid sample having the analyte is applied.

4. The device according to claim 2, further comprising an absorbing pad installed at a lower stream of the membrane to absorb a liquid sample through a capillary phenomenon.

5. The device according to claim 2, wherein the gold ions and the reductant are transferred to the basic strip when the pad for signal amplification is moved to the second position, whereby a signal amplification is caused.

6. The method according to claim 1, or the device according to claim 2, wherein the binding portion (ligand) is at least one selected from the group consisting of protein ligand and nucleic acid (DNA or RNA) molecule sequences; and
the specific binding material is at least one selected from the group consisting of protein, virus phage, nucleic acid molecule Aptamer, and hapten (DNP), which are specifically combined with the binding portion.

7. The method according to claim 1, or the device according to claim 2, wherein the reductant is at least one selected from the group consisting of hydroxylamine and citric acid.

8. The method according to claim 1, or the device according to claim 2, wherein the analyte is selected from the group consisting of DNA, endocrine disruptor, and antigen protein.

9. A measuring device including the lateral flow analysis device of claim 2, and
a reading unit for reading test results of the lateral flow analysis device,
wherein the measuring device comprises a unit for allowing the pad for signal amplification to contact at least one of the bonding pad and the membrane after the sample is inputted.

## Patentansprüche

1. Signalverstärkungsverfahren bei einer Seitenflussanalyse, wobei das Verfahren Folgendes aufweist:
Vorsehen einer flüssigen Probe, die einen Analyt in einem Proben-Pad enthält;
Kombinieren eines Konjugats mit dem Analyt,
wobei das Konjugat einen ersten Antikörper oder ein erstes spezifisches Bindematerial und Goldnanopartikel enthält; wobei der erste Antikörper oder ein erstes spezifisches Bindematerial spezifisch mit einem ersten Epitop oder einem ersten Bindeabschnitt (Liganden) des Analyts kombiniert wird;
Kombinieren des Analyts und kombinierten Konjugats mit einem festgelegten zweiten Antikörper oder zweiten spezifischen Bindematerial über ein zweites Epitop oder einen zweiten Bindeabschnitt (Ligand) des Analyts; und
Zusetzen von Goldionen und einem Reduktionsmittel zum Bewirken von Signalverstärkungsreaktion durch Ausüben von Druck auf das Pad zur Signalverstärkung mit Goldionen und einem Reduktionsmittel, das zwischen einer ersten Position, die nicht in Kontakt mit einem Grundstreifen ist, und einer zweiten Position angeordnet ist, die mit dem Grundstreifen in Kontakt ist, und
wobei das Pad zur Signalverstärkung selektiv mit mindestens einem des Bonding-Pads und der Membran in Kontakt gebracht wird, wenn das Pad zur Signalverstärkung aus der ersten Position zur zweiten Position bewegt wird, durch Druck, der auf das Pad zur Signalverstärkung ausgeübt wird.

2. Seitenflussanalysevorrichtung, aufweisend:
einen Grundstreifen, aufweisend:
ein Bonding-Pad, das ein Konjugat mit einem ersten Antikörper oder einem ersten spezifischen Bindematerial und Goldnanopartikel enthält, wobei der erste Antikörper oder das erste spezifische Bindematerial des Konjugats dazu imstande ist, spezifisch mit einem ersten Epitop oder einem ersten Bindeabschnitt (Ligand) eines Analyts kombiniert zu werden; und
eine Membran mit einer Erkennungsstelle, enthaltend einen festgelegten zweiten Antikörper oder ein zweites spezifisches Bindematerial, wobei der festgelegte zweite Antikörper oder das zweite spezifische Bindematerial dazu imstande ist, spezifisch mit einem zweiten Epitop oder einem zweiten Bindeabschnitt (Ligand) des Analyts kombiniert zu werden;
ein Pad zur Signalverstärkung, das Goldionen und ein Reduktionsmittel enthält, wobei das Pad zwischen einer ersten Position, die nicht in Kontakt mit einem Grundstreifen ist, und einer zweiten Position angeordnet ist, die mit dem Grundstreifen in Kontakt ist, und
wobei das Pad zur Signalverstärkung selektiv mit mindestens einem des Bonding-Pads und der Membran in Kontakt gebracht wird, wenn das Pad zur Signalverstärkung aus der ersten Position zur zweiten Position bewegt wird, durch Druck, der auf das Pad zur Signalverstärkung ausgeübt wird.

3. Vorrichtung nach Anspruch 2, ferner aufweisend ein Proben-Pad, auf das eine flüssige Probe mit dem Analyt aufgebracht ist.

4. Vorrichtung nach Anspruch 2, ferner aufweisend ein Absorptions-Pad, das an einem unteren Strom der Membran eingerichtet ist, zum Absorbieren einer flüssigen Probe durch eine Kapillarerscheinung.

5. Vorrichtung nach Anspruch 2, wobei die Goldionen und das Reduktionsmittel auf den Grundstreifen überführt werden, wenn das Pad zur Signalverstärkung zur zweiten Position bewegt wird, wodurch eine Signalverstärkung bewirkt ist.

6. Verfahren nach Anspruch 1 oder Vorrichtung nach Anspruch 2, wobei der Bindeabschnitt (Ligand) mindestens eines ist, das aus der Gruppe ausgewählt ist, die aus Proteinligand und Nucleinsäure- (DNA- oder RNA-) Molekülsequenzen besteht; und
wobei das spezifische Bindematerial mindestens eines ist, das aus der Gruppe ausgewählt ist, die aus Protein, Virusphage, Nucleinsäuremolekül-Aptamer und Hapten (DNP) besteht, die spezifisch mit dem Bindeabschnitt kombiniert werden.

7. Verfahren nach Anspruch 1 oder Vorrichtung nach Anspruch 2, wobei das Reduktionsmittel mindestens eines ist, das aus der Gruppe ausgewählt ist, die aus Hydroxylamin und Zitronensäure besteht.

8. Verfahren nach Anspruch 1 oder Vorrichtung nach Anspruch 2, wobei der Analyt aus der Gruppe ausgewählt ist, die aus DNA, endokrinem Disruptor und Antigenprotein besteht.

9. Messgerät, das die Seitenflussanalysevorrichtung gemäß Anspruch 2 und
eine Leseeinheit zum Lesen von Testergebnissen der Seitenflussanalysevorrichtung enthält,
wobei das Messgerät eine Einheit zum Ermöglichen aufweist, dass das Pad zur Signalverstärkung mindestens eines des Bonding-Pads und der Membran kontaktiert, nachdem die Probe eingegeben wurde.

## Revendications

1. Procédé d'amplification du signal dans une analyse sur membrane, le procédé comprenant les étapes suivantes :
se procurer un échantillon liquide comportant un analyte dans un tampon à échantillon ;
combiner un conjugué avec l'analyte,
le conjugué comportant un premier anticorps ou un premier liant spécifique et des nanoparticules d'or ; le premier anticorps ou un premier liant spécifique étant spécifiquement combiné avec un premier épitope ou une première partie de liaison (ligand) de l'analyte ;
combiner l'analyte et le conjugué combiné avec un deuxième anticorps fixé ou un deuxième liant spécifique par le biais d'un deuxième épitope ou d'une deuxième partie de liaison (ligand) de l'analyte ; et
ajouter des ions or et un réducteur pour provoquer une réaction d'amplification du signal en appliquant une pression au tampon pour amplification du signal comportant des ions or et un réducteur qui est disposé entre une première position qui n'est pas en contact avec une bandelette basique et une deuxième position qui est en contact avec la bandelette basique, et
dans lequel le tampon pour amplification du signal est sélectivement mis en contact avec le tampon de liaison et/ou la membrane, quand le tampon pour amplification du signal est déplacé de la première position à la deuxième position par une pression appliquée au tampon pour amplification du signal.

2. Dispositif d'analyse sur membrane comprenant :
une bandelette basique comprenant :
un tampon de liaison comportant un conjugué ayant un premier anticorps ou un premier liant spécifique et des nanoparticules d'or, le premier anticorps ou le premier liant spécifique du conjugué étant susceptible d'être spécifiquement combiné avec un premier épitope ou une première partie de liaison (ligand) d'un analyte ; et
une membrane ayant un site de détection comportant un deuxième anticorps fixé ou un deuxième liant spécifique, le deuxième anticorps fixé ou le deuxième liant spécifique étant susceptible d'être spécifiquement combiné avec un deuxième épitope ou une deuxième partie de liaison (ligand) de l'analyte ;
un tampon pour amplification du signal comportant des ions or et un réducteur, le tampon pour amplification du signal étant disposé entre une première position qui n'est pas en contact avec la bandelette basique et une deuxième position qui est en contact avec la bandelette basique, et
dans lequel le tampon pour amplification du signal est sélectivement mis en contact avec le tampon de liaison et/ou la membrane, quand le tampon pour amplification du signal est déplacé de la première position à la deuxième position par une pression appliquée au tampon pour application du signal.

3. Dispositif selon la revendication 2, comprenant en outre un tampon à échantillon auquel un échantillon liquide ayant l'analyte est appliqué.

4. Dispositif selon la revendication 2, comprenant en outre un tampon absorbant installé au niveau d'un courant inférieur de la membrane pour absorber un échantillon liquide par un phénomène capillaire.

5. Dispositif selon la revendication 2, dans lequel les ions or et le réducteur sont transférés à la bandelette basique quand le tampon pour amplification du signal est déplacé jusqu'à la deuxième position, une amplification du signal étant ainsi provoquée.

6. Procédé selon la revendication 1, ou dispositif selon la revendication 2, la partie de liaison (ligand) étant au moins un élément choisi dans le groupe constitué par un ligand protéique et des séquences de molécule d'acide nucléique (ADN ou ARN) ; et
le liant spécifique est au moins un élément choisi dans le groupe constitué par une protéine, un phage viral, un aptamère de molécule d'acide nucléique, et un haptène (DNP), qui sont spécifiquement combinés avec la partie de liaison.

7. Procédé selon la revendication 1, ou dispositif selon la revendication 2, le réducteur étant au moins un élément choisi dans le groupe constitué par l'hydroxylamine et l'acide citrique.

8. Procédé selon la revendication 1, ou dispositif selon la revendication 2, l'analyte étant choisi dans le groupe constitué par un ADN, un perturbateur endocrinien, et une protéine antigénique.

9. Dispositif de mesure comportant le dispositif d'analyse sur membrane de la revendication 2, et
une unité de lecture destinée à lire des résultats d'essai du dispositif d'analyse sur membrane,
le dispositif de mesure comprenant une unité destinée à permettre au tampon pour amplification du signal d'entrer en contact avec le tampon de liaison et/ou la membrane après que l'échantillon a été introduit.
